(19) Europäisches Patentamt
European Patent Office
Office européen des brevets

(11) Veröffentlichungsnummer: **0 220 516 B1**

(12) # EUROPÄISCHE PATENTSCHRIFT

(45) Veröffentlichungstag der Patentschrift: **04.01.89**

(51) Int. Cl.⁴: **C07C 51/60**, C07C 63/30 // B01J27/14

(21) Anmeldenummer: **86113393.2**

(22) Anmeldetag: **29.09.86**

(54) **Verfahren zur Herstellung von aromatischen Carbonsäurechloriden.**

(30) Priorität: **09.10.85 DE 3535984**

(43) Veröffentlichungstag der Anmeldung: **06.05.87 Patentblatt 87/19**

(45) Bekanntmachung des Hinweises auf die Patenterteilung: **04.01.89 Patentblatt 89/1**

(84) Benannte Vertragsstaaten: **BE CH DE FR GB IT LI**

(56) Entgegenhaltungen:
**EP-A- 0 022 484**
**EP-A- 0 024 286**
**DE-A- 2 321 122**
**FR-A- 1 080 261**

**CHEMICAL ABSTRACTS, Band 54, nr. 6, 25. März 1960, Spalte 5425, e-f, Columbus Ohio, US: L.HORNER et al.: " Phosphorus organic compounds. XVII. Reactions with triphenylphosphine dihalides; the reaction path of halogen introduction or dehydration by phosphorus halides", & Ann. 62, 26-34 (1959) 000**

(73) Patentinhaber: **BAYER AG, Konzernverwaltung RP Patentabteilung, D-5090 Leverkusen 1 Bayerwerk(DE)**

(72) Erfinder: **Buysch, Hans-Josef, Dr., Brandenburger Strasse 28, D-4150 Krefeld(DE)**

**EP 0 220 516 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein Verfahren zur Herstellung von aromatischen Carbonsäurechloriden durch Behandlung von Alkylestern aromatischer Carbonsäuren mit Phosgen in Gegenwart von Phosphorverbindungen.

Aus US 2 865 959 ist es bekannt, aus Estern aromatischer Carbonsäuren durch Umsetzung mit Chlor in Gegenwart von Metallchloriden, wie Eisentrichlorid, Antimontrichlorid oder Zinkchlorid aromatische Carbonsäurechloride herzustellen. Neben den gewünschten Säurechloriden entstehen dabei Chlorwasserstoff und eine Vielzahl von Umwandlungsprodukten aus den Alkoholkomponenten der Ester. Solche Umwandlungsprodukte sind beispielsweise Aldehyde, Ketone, Alkohole, Olefine, Alkylmono- und Alkyldichloride: Methylester liefern als erstes Nebenprodukt Formaldehyd.

Viele dieser Umwandlungsprodukte sind unter den Reaktionsbedingungen nicht stabil. Insbesondere Aldehyde und Ketone verhalten sich gegenüber Chlor sehr reaktiv und liefern neben zusätzlichem Chlorwasserstoff weitere aggressive, toxische und schleimhautreizende Produkte. In Gegenwart des abgespaltenen Chlorwasserstoffs können Carbonylverbindungen auch Additions- und Kondensationsreaktionen, beispielsweise unter Wasseraustritt, eingehen. Insgesamt erhält man also eine Fülle verschiedener, nur schwer trennbarer Stoffe, die entweder im Reaktionsgemisch verbleiben oder mit dem Abgasstrom ausgetragen werden und in jedem Falle nur mit Hilfe umfangreicher Aufarbeitungsmethoden entfernt und unschädlich gemacht werden können.

Aus DE-AS 2 158 551 ist es bekannt, durch Chlorieren von Polyestern aromatischer Dicarbonsäuren die zugehörigen Dicarbonsäuredichloride zu gewinnen. Auch bei diesem Verfahren muß mit den oben erwähnten Schwierigkeiten gerechnet werden.

In beiden erwähnten Verfahren muß weiterhin mit einer Kernchlorierung gerechnet werden, insbesondere wenn mit den erwähnten Metallchlorid-Katalysatoren gearbeitet wird. Die Behandlung des in beiden Verfahren in großen Mengen entstehenden Chlorwasserstoffe ist eine weitere Belastung der genannten Verfahren.

In DE-OS 2 321 122, Beispiel 5, wird die Umsetzung von Terephthalsäuremonomethylester bei 100°C mit Phosgen in Gegenwart von Trimethylphosphinoxid beschrieben. Bei dieser Temperatur tritt jedoch keine Esterspaltung ein, es wird nur die freie Carboxylgruppe halogeniert.

In FR 1 080 261 wird die Umsetzung von Estern mit Phosgen beschrieben, wobei neben korrosiven sauren Beschleunigern auch Schwermetalle, wie Eisen oder Kupfer, auch Basen, wie Pyridin, eingesetzt werden können. Lt. Beispiel 1 dieser FR-PS ist zur Umsetzung des n-Butylesters der (aromatischen) Benzoesäure die Kombination des basischen Pyridins mit dem sauren Phosphortrichlorid erforderlich. Pyridin geht jedoch in Gegenwart von Phosgen bei hohen Temperaturen in gefärbte Verbindungen über, die nur schwierig zu entfernen sind.

Es wurde ein Verfahren zur Herstellung von aromatischen Carbonsäurechloriden aus Alkylestern aromatischer Carbonsäuren gefunden, das dadurch gekennzeichnet ist, daß man die Alkylester aromatischer Carbonsäuren bei 130–270°C in Gegenwart von Organophosphorverbindungen der Formel

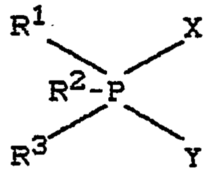

(I),

worin

$R^1$, $R^2$ und $R^3$ unabhängig voneinander Alkyl, Cycloalkyl, Aralkyl oder Aryl bedeuten können, wobei $R^1$ und $R^2$ auch gemeinsam mit dem Phosphoratom einen 5- oder 6gliedrigen Ring bilden können und weiterhin $R^1$ auch Halogen bedeuten kann, und

X und Y für gleiches oder verschiedenes Halogen stehen, aber auch gemeinsam doppelt gebundenen Sauerstoff, doppelt gebundenen Schwefel, doppelt gebundenen Stickstoff $=N-R^4$, worin $R^4$ Alkyl, Cycloalkyl, Aralkyl, Aryl, Acyl, Aryloxycarbonyl, Dialkylkphosphonyl oder Diarylphosphonyl, bevorzugt Alkyl, Aryl oder Acyl bedeutet oder ein Elekronenpaar bedeuten können und weiterhin X Alkyl, Aralkyl, Aryl oder Allyl bedeutet, wenn gleichzeitig Y eine positive Teilladung am Phosphoratom darstellt, wobei in diesem Falle ein negatives Gegenion vorhanden ist, das gegebenenfalls auch als Substituent auf X angeordnet sein kann, wenn X die Bedeutung Alkyl, Aralkyl, Aryl oder Allyl hat, und gegebenenfalls in Gegenwart eines inerten Lösungsmittels mit Phosgen behandelt.

Die Reaktion des erfindungsgemäßen Verfahrens kann allgemein wie folgt formuliert werden:

$$\text{Aryl–CO–O–Alkyl} + COCl_2 \rightarrow \text{Aryl–CO–Cl} + CO_2 + \text{Cl–Alkyl}.$$

Neben dem gewünschten Säurechlorid entsteht Kohlendioxid und das unter den Reaktionsbedingungen stabile zugehörige Alkylchlorid, das in hoher Ausbeute gewonnen wird und für eine Reihe anderer technischer Prozesse eingesetzt werden kann.

Als Aryl in der obigen Formulierung wird ein von einem aromatischen System abgeleiteter Rest verstanden. Solche aromatischen Systeme sind beispielsweise Benzol, Naphthalin, Anthracen, Phenanthren oder Systeme, in denen zwei Benzolkerne durch ein Brückenglied verbunden sind, wobei das

Brückenglied beispielsweise eine einfache Bindung, Sauerstoff, Schwefel, die Sulfongruppe $-SO_2-$, die Carbonylgruppe $-CO-$, ein $C_1$–$C_6$-Alkylidenrest oder ein $C_5$–$C_7$-Cycloalkylidenrest darstellt. Beispiele für die zuletzt genannten, durch Brückenglieder gebildete Systeme sind: Diphenyl, Diphenylether, Diphenylthioether, Diphenylsulfon, Benzophenon, Diphenylmethan, 2,2-Diphenylpropan und 1,1-Diphenylcyclohexan.

Die aus den genannten Systemen hervorgehenden Arylreste können substituiert sein, beispielsweise durch Halogen, wie Fluor, Chlor, Brom, Jod, bevorzugt Fluor, Chlor oder Brom, besonders bevorzugt Chlor;
$C_1$–$C_4$-Alkyl, wie Methyl, Ethyl, Propyl, Isopropyl, Butyl, Isobutyl, bevorzugt Methyl oder Ethyl; $C_1$–$C_4$-Alkoxy, wie Methoxy, Ethoxy, Propyloxy, Isopropyloxy, Butyloxy oder Isobutyloxy, bevorzugt Methoxy oder Ethoxy; Nitro; Cyano.

Der Arylrest kann einen oder mehrere der genannten Substituenten, gegebenenfalls auch verschiedene der genannten Arten, tragen.

Der Arylrest trägt ferner eine oder mehrere Carbonsäurefunktionen, so daß dem Alkylester aromatischer Carbonsäuren gegebenenfalls eine aromatische Monocarbonsäure, Dicarbonsäure oder Polycarbonsäure zugrunde liegen kann.

Beispiele für aromatische Carbonsäuren in den erfindungsgemäß umzusetzenden Estern sind: Benzoesäure, o-, m-, p-Methylbenzoesäure, o-, m-, p-Chlorbenzoesäure, tert.-Butylbenzoesäure, Methoxybenzoesäure, Fluorbenzoesäure, Brombenzoesäure, Nitrobenzoesäure, Dimethylbenzoesäuren, Dichlorbenzoesäuren, Phthalsäure, Isophthalsäure, Terephthalsäure, Naphthalindicarbonsäure, Diphenyldicarbonsäure, Diphenylether-dicarbonsäure, Isophthalsäure, Benzoltricarbonsäuren, Benzoltetracarbonsäure, insbesondere Benzoesäure, Iso- und Terephthalsäure.

Als Alkyl in der obigen allgemeinen Formulierung sei ein offenkettiges oder cyclisches Alkyl verstanden.

Offenkettiges Alkyl sei beispielsweise ein $C_1$–$C_{18}$-, bevorzugt $C_1$–$C_8$-, besonders bevorzugt $C_1$–$C_4$-Alkyl, wie Methyl, Ethyl, Propyl, Isopropyl, Butyl, Isobutyl, Hexyl, Octyl, Dodecyl, Palmityl oder Stearyl.

Cyclisches Alkyl sei beispielsweise $C_4$–$C_7$-, bevorzugt $C_5$–$C_6$-Cycloalkyl, wie Cyclobutyl, Cyclopentyl, Cyclohexyl, Cycloheptyl, Methylcyclopentyl oder Methylcyclohexyl.

Die genannten Alkylreste können substituiert sein, beispielsweise durch $C_1$–$C_4$-Alkyl oder Halogen in der oben beschriebenen Weise, weiterhin durch Phenyl, das seinerseits mit den oben für Aryl beschriebenen Substituenten substituiert sein kann. Alkyl kann ferner durch die Hydroxylgruppe substituiert sein, wenn deren Umwandlung in einen Chlorsubstituenten während des erfindungsgemäßen Verfahrens zugelassen wird.

Der Alkylrest in der obigen allgemeinen Formulierung umfaßt weiterhin auch Alkyliden- oder Cycloalkylidenreste, so daß also der den erfindungsgemäß umzusetzenden Alkylestern aromatischer Carbonsäuren zugrunde liegende Alkohol neben einem Monoalkohol auch ein Di- oder Trialkohol sein kann.

Nach dem erfindungsgemäßen Verfahren können demnach folgende Alkylester aromatischer Carbonsäuren umgesetzt werden: Monoester aus einer aromatischen Monocarbonsäure und einem Monoalkanol; Diester aus einer aromatischen Dicarbonsäure und zwei Molekülen eines Monoalkanols oder gegebenenfalls zweier verschiedener Monoalkanole; Diester aus einem Alkandiol und zwei Molekülen einer aromatischen Carbonsäure oder gegebenenfalls zweier verschiedener aromatischer Carbonsäuren; Triester oder noch höhere Ester aus aromatischen Tricarbonsäuren oder noch höheren Carbonsäuren und Alkanolen in der genannten Art; Triester aus Alkantriolen und aromatischen Carbonsäuren in der genannten Art; schließlich Oligoester oder Polyester von Di- oder Polycarbonsäuren mit Alkandiolen oder -polyolen. Als Beispiel für den zuletzt genannten Fall lassen sich Abfälle aus der Polyesterfabrikation und -verarbeitung, insbesondere Polyethylen- und Polybutylenterephthalate dem erfindungsgemäßen Verfahren unterwerfen, wobei im Falle des Polybutylenphthalats Terephthalsäuredichlorid und 1,4-Dichlorbutan gewonnen werden. In jedem der genannten Fälle können alle im Reaktionsgemisch vorhandenen Estergruppen im erfindungsgemäßen Sinne reagieren. Nicht vollständig umgesetzte Estergruppen, beispielsweise nicht vollständig abgebaute Polyester, können einem Folgeansatz zur erneuten erfindungsgemäßen Umsetzung zugegeben werden.

Die Organophosphorverbindungen der Formel (I) umfassen also beispielsweise Organophosphine (Formel II), Organophosphinoxide (III), Organophosphinsulfide (IV), Organophosphoniumsalze (V), Organophosphorbetaine (VI), Dihalogen-organophosphine (VII) und N-substituierte Organophosphinimine (VIII).

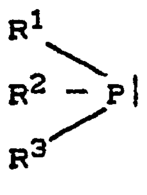 (II), 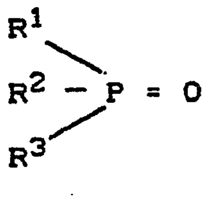 (III), 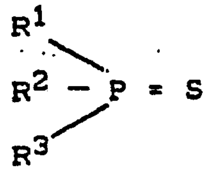 (IV),

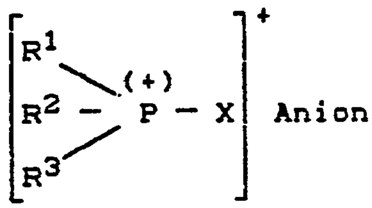 (V), 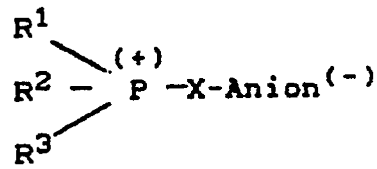 (VI)

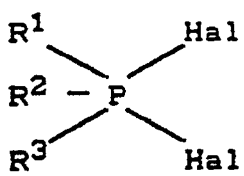 (VII), 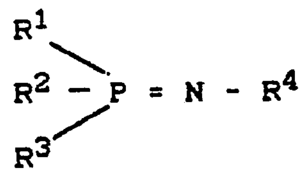 (VIII)

In den Formeln (I) bis (VIII) haben Alkyl, Aryl und Halogen die zuvor genannte Bedeutung, wobei Alkyl auch Cycloalkyl einschließt. Als Aralkyl sei beispielsweise Benzyl, Phenylethyl, Naphthylmethyl oder Naphthylethyl, bevorzugt Benzyl genannt.

Die Reste $R^1$, $R^2$, $R^3$ und X in der Bedeutung von Alkyl, Aryl oder Aralkyl können in der oben geschilderten Weise substituiert sein. $R^1$ und $R^2$ können gemeinsam auch Tetramethylen oder Pentamethylen bedeuten und gemeinsam mit dem Phosphoratom einen 5- oder 6gliedrigen Ring bilden.

Als Anion in (V) oder (VI) seien beispielsweise genannt: Chlorid, Bromid, Hydroxid, Sulfat (1 Äquivalent), Sulfonat, Carboxylat.

Als Beispiele für erfindungsgemäß einsetzbare Organophosphorverbindungen seien genannt: Diphenylchlorphosphin, Tributylphosphin, Tris(cyanethyl)-phosphin, Dicyclohexyldodecylphosphin, Triphenylphosphin, Tris-(p-chlor-phenyl)-phosphin, 1-Methyl-phospholin, Triphenyl-benzyl-phosphoniumchlorid, Tributyl-allyl-phosphoniumbromid, Triphenylphosphincarbomethoxy-methylen, β-Triphenylphosphoniumpropionat, Phenyloxy-trimethylphosphoniumchlorid, β-Triphenylphosphonium-ethylsulfonat, Triphenylphosphin-hydroxid-chlorid, Triethyl-carbonyloxyphenyl-chlorphosphin, 1,1-Dichlortriphenylphosphin, 1,1-Dibrom-tris(cyanethyl)-phosphin, 1-Phenyl-phospholin(3)-1,1-dichlorid, Dimethylcyclohexylphosphinoxid, Tris(chlorpropyl)-phosphinoxid, Dimethyl-phenylphosphinoxid, Tris-(p-cyanophenyl)-phosphinoxid, Triphenylphosphinsulfid, Trimethylphosphinsulfid, 1-Methyl-phospholin(3)-1-oxid, 1-Phenyl-phospholin(3)-1-oxid, Tris(hydroxyethyl)-phosphin, Triethyl-N-acetyl-phosphinimin und Triphenyl-N-phenyl-phosphinimin.

In bevorzugter Weise werden Organophosphorverbindungen der Formeln (II), (III) und (VI) eingesetzt; besonders bevorzugt werden Organophosphorverbindungen der Formeln (II) und (III) eingesetzt. Solche Verbindungen sind beispielsweise Tributylphosphin, Tris-(cyanethyl)-phosphin, Triphenylphosphin, Tris-(4-chlorphenyl)-phosphin, Triphenylphosphinoxid, β-Triphenyl-phosphoniumpropionat, insbesondere Triphenylphosphin und Triphenylphosphinoxid.

Anstelle einer Organophosphorverbindung kann auch ein Gemisch mehrerer eingesetzt werden. Ebenso kann die Organophosphorverbindung oder ein Gemisch mehrerer, die bei der Aufarbeitung des Reaktionsgemisches als Destillationsrückstand verbleiben, für einen weiteren Ansatz neu verwendet werden.

Die Menge an Katalysator beträgt 0,01–15 Gew.-%, bevorzugt 0,1–10 Gew.-%, besonders bevorzugt 0,3–7 Gew.-%, alles bezogen auf die Menge des eingesetzten Alkyesters einer aromatischen Carbonsäure.

Das erfindungsgemäße Verfahren kann mit oder ohne Lösungsmittel durchgeführt werden. Als Lösungsmittel sind unter den Reaktionsbedingungen inerte und möglichst leicht abtrennbare Stoffe geeignet, beispielsweise aliphatische und aromatische Kohlenwasserstoffe, aliphatische und aromatische Halogenkohlenwasserstoffe, aliphatische und aromatische Nitrile, wie Testbenzin, Toluol, Xylol, Methylenchlorid, Trichlorethylen, Dichlorbenzol, Chlornaphtalin, Diphenylether und Benzonitril. Weiterhin kommen als unter den Reaktionsbedingungen inerte und leicht abtrennbare Lösungsmittel Säurechloride in Betracht, insbesondere das bei der Durchführung des erfindungsgemäßen Verfahrens entstehende aromatische Carbonsäurechlorid.

Das erfindungsgemäße Verfahren wird bei einer Temperatur von 130–270°C, bevorzugt 150–250°C, durchgeführt.

Das erfindungsgemäße Verfahren kann in einem weiten Druckbereich durchgeführt werden, beispielsweise bei 0,1–50 bar, bevorzugt 0,5–30 bar, besonders bevorzugt 0,8–10 bar. Im allgemeinen ist die Anwendung eines erhöhten Druckes dann erforderlich, wenn ein niedrig siedendes Substrat oder Lösungsmittel eingestzt wird.

Phosgen ist im erfindungsgemäßen Verfahren zur weitgehenden Vervollständigung der Umsetzung in einer Menge von mindestens 1 Mol pro Estergruppe erforderlich. Phosgen kann jedoch auch in einem Überschuß von bis zu 100 Mol, bevorzugt bis zu 50 Mol pro Estergruppe eingesetzt werden. Der nicht verbrauchte Überschuß an Phosgen kann in einem Folgeeinsatz erneut verwendet werden. Das Phosgen kann flüssig oder gasförmig in das Reaktionsgefäß eingespeist werden. Flüssiges Phosgen kann grundsätzlich auch als Lösung in einem der genannten Lösungsmittel benutzt werden; ebenso kann gasförmiges Phosgen im Gemisch mit einem Inertgas, wie Stickstoff, Argon oder Kohlendioxid verwendet werden.

Das erfindungsgemäße Verfahren kann diskontinuierlich oder kontinuierlich durchgeführt werden. In beiden Fällen kann der Einsatz von gasförmigem Phosgen vorteilhaft in einer Blasensäulenapparatur erfolgen. Bei kontinuierlicher Verfahrensweise wird beispielsweise das phosgenhaltige Abgas der ersten Blasensäule zweckmäßig durch weitere nachgeschaltete Blasensäulen geleitet. Die letzte Blasensäule wird dann mit frischem Carbonsäureester beschickt und der Überlauf einer Blasensäule wird am Fuß der nächsten eingeleitet. Auf diese Weise erhält man am Kopf der letzten Blasensäule ein praktisch phosgenfreies und im wesentlichen aus Kohlendioxid bestehendes Abgas, das nach einfacher Reinigung von mitgerissenen Spuren Carbonsäurechlorid, Alkylchlorid und gegebenenfalls Lösungsmittel entweder als $CO_2$ verwendet oder in die Atmosphäre abgegeben werden kann.

Beispiel 1

Ein Gemisch von 150 g Terephthalsäure-di-n-hexylester und 7,5 g Triphenylphosphinoxid wurden in einer 250 ml fassenden zylindrischen Apparatur aufgeschmolzen und von unten durch eine Fritte bei Normaldruck mit Phosgen begast. Die Temperatur wurde zwischen 170 und 190°C gehalten. Das Abgas wurde durch eine auf ca. 10°C gehaltene Kühlfalle geleitet. Nach 25 h enthielt das Reaktionsgemisch neben nicht umgesetztem Terephthalsäure-di-n-hexylester 21,2 g Terephthalsäure-n-hexylesterchlorid und 52,6 g Terephthalsäuredichlorid. In der Kühlfalle befanden sich 64,6 g n-Hexylchlorid (91% der theoretischen Menge, bezogen auf das entstandene Säureesterchlorid und Säuredichlorid).

Beispiel 2

Ein Gemisch von 150 g Terephthalsäuredimethylester und 7 g Triphenylphosphinoxid wurde wie in Beispiel 1 mit Phosgen begast. Nach 5 h enthielt das Reaktionsgemisch nur noch 1,3% Terephthalsäuredimethylester und 1,3% Terephthalsäuremethylesterchlorid. Nach 6–7 h war der Umsatz vollständig, und man erhielt Terephthalsäuredichlorid (>99%ig).

Beispiel 3

Eine Lösung von 100 g Polybutylenterephthalat mit einem Molgewicht von etwa 25,000 und 5 g Triphenylphosphinoxid in 300 g o-Dichlorbenzol wurde, wie in Beispiel 1 beschrieben, 10 h bei 170–175°C mit Phosgen begast. Die anfänglich viskose Lösung wurde rasch dünnflüssiger. Die Kühlfalle und das Reaktionsgemisch enthielten neben dem o-Dichlorbenzol, 1,4-Dichlorbutan, das aus dem Butylenrest entstanden ist. Beim Aufdestillieren des Reaktionsgemisches gingen nach dem o-Dichlorbenzol und 1,4-Dichlorbutan 33 g Terephthalsäuredichlorid und bei 180–230°C/1–2 mbar 38 g eines Gemisches nicht näher charakterisierter chlorhaltiger Spaltprodukte über, die einem Folgeansatz zugesetzt wurden, dort die Ausbeute an 1,4-Dichlorbutan und Terephthalsäuredichlorid erhöhten und somit in diese Stoffe umsetzbar sind.

**Patentansprüche**

1. Verfahren zur Herstellung von aromatischen Carbonsäurechloriden aus Alkylestern aromatischer Carbonsäuren, dadurch gekennzeichnet, daß man die Alkylester aromatischer Carbonsäuren bei 130–270°C in Gegenwart von Organophosphorverbindungen der Formel

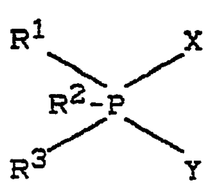

(I),

worin

$R^1$, $R^2$ und $R^3$ unabhängig voneinander Alkyl, Cycloalkyl, Aralkyl oder Aryl bedeuten können, wobei $R^1$ und $R^2$ auch gemeinsam mit dem Phosphoratom einen 5- oder 6gliedrigen Ring bilden können und weiterhin $R^1$ auch Halogen bedeuten kann, und

X und Y für gleiches oder verschiedenes Halogen stehen, aber auch gemeinsam doppelt gebundenen Stickstoff =N–$R^4$, worin $R^4$ Alkyl, Cycloalkyl, Aralkyl, Aryl, Acyl, Aryloxycarbonyl, Dialkylkphosphonyl oder Diarylphosphonyl, bevorzugt Alkyl, Aryl oder Acyl bedeutet oder ein Elektronenpaar bedeuten können und weiterhin X Alkyl, Aralkyl, Aryl oder Allyl bedeutet, wenn gleichzeitig Y eine positive Teilladung am Phosphoratom darstellt, wobei in diesem Falle ein negatives Gegenion vorhanden ist, das gegebenenfalls auch als Substituent auf X angeordnet sein kann, wenn X die Bedeutung Alkyl, Aralkyl, Aryl oder Allyl hat, und gegebenenfalls in Gegenwart eines inerten Lösungsmittels mit Phosgen behandelt.

2. Verfahren nach Anspruch 1, dadurch gekennzeichnet, daß als Organophosphorverbindung ein Organophosphin oder Organophosphinoxid eingesetzt wird.

3. Verfahren nach Anspruch 1 und 2, dadurch gekennzeichnet, daß als Organophosphorverbindung Triphenylphosphinoxid eingesetzt wird.

4. Verfahren nach Ansprüchen 1 bis 3, dadurch gekennzeichnet, daß bei 150–250°C gearbeitet wird.

5. Verfahren nach Anspruch 1 bis 4, dadurch gekennzeichnet, daß 1–100 Mol Phosgen pro Äquivalent Ester eingesetzt werden.

6. Verfahren nach Anspruch 1 bis 5, dadurch gekennzeichnet, daß als Alkylester einer aromatischen Carbonsäure Polyalkylenterephthalat in Gegenwart eines Lösungsmittels eingesetzt wird.

7. Verfahren nach Anspruch 1 bis 6, dadurch gekennzeichnet, daß die Umsetzung kontinuierlich in einer Blasensäulenapparatur durchgeführt wird.

## Claims

1. Process for the preparation of aromatic carboxylic acid chlorides from alkyl esters of aromatic carboxylic acids, characterized in that the alklyl esters of aromatic carboxylic acids are treated with phosgene at 130–270°C in the presence of organophosphorus compounds of the formula

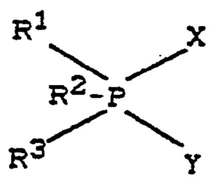

(I),

wherein

$R^1$, $R^2$ and $R^3$ independently of one another can denote alkyl, cycloalkyl, aralkyl or aryl, or wherein $R^1$ and $R^2$, together with th phosphorus atom, can also form a 5-membered or 6-membered ring, and futhermore $R^1$ can also denote halogen, and

X and Y represent inditical of different halogen, but can also together denote double-bonded oxygen, double-bonded sulphur, double-bonded nitrogen =N–$R^4$, wherein $R^4$ denotes alkyl, cycloalkyl, aralkyl, aryl, acyl, aryloxycarbonyl, dialkylphosphonyl or diarylphosphonyl, preferably alkyl, aryl or acyl, or an electron pair, and furthermore X denotes alkyl, aralkyl, aryl or allyl, if Y simultaneously represents a positive partial charge on the phosphorus atom, a negative counter ion which, if appropriate, can also be allocated to X as a substituent if X denotes alkyl, aralkyl, aryl or allyl, being present in this case,

and, if appropriate, in the presence of an inert solvent.

2. Process according to claim 1, characterized in that an organophosphine or organophosphine oxide is employed as the organophosphorus compound.

3. Process according to claims 1 and 2, characterized in that triphenylphosphine oxide is employed as the organophosphorus compound.

4. Process according to claims 1 to 3, characterized in that it is carried out at 150–250°C.

5. Process according to claims 1 to 4, characterized in that 1–100 mol of phosgene are used per equivalent of ester.

6. Process according to claims 1 to 5, characterized in that polyalkylene terephthalate in the presence of a solvent is employed as the alkyl ester of an aromatic carboxylic acid.

7. Process according to claims 1 to 6, characterized in that the reaction is carried out continuously in a bubble column apparatus.

## Revendications

1. Procédé pour préparer des chlorures d'acides carboxyliques aromatiques à partir d'esters alkyliques d'acides carboxyliques aromatiques, procédé caractérisé en ce qu'on traite par du phosgène les esters alkyliques d'acides carboxylique aromatiques à 130–270°C, en présence de composés organiques du phosphore, de formule:

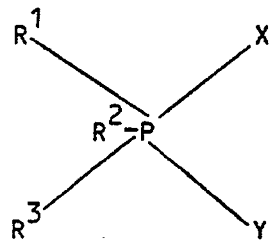

(I),

dans laquelle

$R^1$, $R^2$ et $R^3$ représentent, indépendamment l'un de l'autre, chacun un groupe alkyle, cycloalkyle, aralkyle ou aryle, R1 et $R^2$ pouvant former egalement, avec l'atome de phosphore, un noyau pentagonal ou hexagonal et, en outre, $R^1$ pouvant également représenter un atome d'halogène, et

X et Y représentent un halogène, identiques ou différents, mais peuvent représenter ensemble aussi de l'oxygène doublement lié, du soufre doublement lié, de l'azote doublement lié =R–$R^4$, où $R^4$ représente un groupe alkyle, cycloalkyle, aralkyle, aryle, arcyle, aryloxycarbonyle, dialkylphosphonyle ou diarylphosphonyle, de préférence un groupe alkyle, aryle ou acyle ou une paire d'électrons et, en outre, X représente un groupe alkyle, aralkyle, aryle ou allyle quand, en même temps, Y représente une charge partielle positive sur l'atome de phosphore, et, dans ce cas, il y a présence d'un ion antagoniste négatif, qui peut éventuellement aussi être fixé comme substituant sur X, quand X représente un groupe alkyle, aralkyle, aryle ou allyle, en opérant éventuellement en présence d'un solvant inerte.

2. Procédé selon la revendication 1, caractérisé en ce qu'on utilise, comme composé organique du phosphore, une organophosphine ou un oxyde d'organophosphine.

3. Procédé selon les revendications 1 et 2, caractérisé en ce qu'on utilise, comme composé organique du phosphore, l'oxyde de triphénylphosphine.

4. Procédé selon les revendications 1 à 3, caractérisé en ce qu'on travaille entre 150 et 250°C.

5. Procédé selon les revendications 1 à 4, caractérisé en ce qu'on utilise 1 à 100 mol de phosgène par équivalent d'ester.

6. Procédé selon les revendications 1 à 5, caractérisé en ce qu'on utilise, comme ester alkylique d'un acide carboxylique aromatique, du poly(téréphtalate d'alkylène) en présence d'un solvant.

7. Procédé selon les revendications 1 à 6, caractérisé en ce qu'on conduit la réaction en continu dans un appareillage comportant des colonnes à boules.